# EUROPEAN PATENT APPLICATION

(11) **EP 4 809 944 A1**
(43) Date of publication of application: **23.09.2026**
(21) Application number: 23958688.6
(22) Date of filing: 24.11.2023
(51) Int. Cl.: A61B 17/3207, A61B 90/00

(54) **ROTARY GRINDING APPARATUS, ROTARY GRINDING DEVICE, ROTARY GRINDING ROTATIONAL SPEED ACQUISITION METHOD AND APPARATUS, COMPUTER DEVICE, STORAGE MEDIUM, AND COMPUTER PROGRAM PRODUCT**

(30) Priority: 17.11.2023 CN 202311545694
(71) Applicant: Shanghai Microport Rotapace Medtech Co., Ltd., Shanghai 201203 (CN)
(72) Inventor: YU, Junyuan, Shanghai 201203 (CN); JI, Xiaofei, Shanghai 201203 (CN); YAO, Yingzhong, Shanghai 201203 (CN); YUE, Bin, Shanghai 201203 (CN)
(74) Representative: Murgitroyd & Company
(86) International application number: PCT/CN2023/133813
(87) International publication number: WO 2025/102419

(57) **Abstract**

Disclosed are a rotary grinding device, a rotary grinding apparatus, a method and an apparatus for obtaining a rotational grinding speed, a computer device, a storage medium, and a computer program product. During rotation of a rotating member (200) with a drive shaft (100), a trigger signal is generated by a trigger mark on the rotating member (200) directly sleeved over the drive shaft (100). Signal time differences each between two consecutive trigger signals are obtained. When the number of obtained signal time differences reaches a target number, a rotational speed of the drive shaft (100) is determined based on all the signal time differences, and a rotational speed waveform representing a variation trend of a rotational speed of a burr is displayed based on a plurality of rotational speeds. As such, no internal structural adjustment is required due to additional disposition of an optical fiber probe that would otherwise affect operation accuracy. Under cooperation of a detection member and a controller, rotational speed detection can be performed at any time, to accurately obtain a variation of the rotational grinding speed, thereby improving operational flexibility and usability.

## Description

### CROSS-REFERENCE TO RELATED APPLICATIONS

This application claims priority to Chinese Patent Application No. 202311545694.2, filed on November 17, 2023, and entitled "ROTARY GRINDING DEVICE, ROTARY GRINDING APPARATUS, AND METHOD FOR OBTAINING ROTATIONAL GRINDING SPEED", the disclosure of which is hereby incorporated herein by reference in its entirety.

### TECHNICAL FIELD

The present application relates to the technical field of rotational atherectomy catheters, and in particular, to a rotary grinding device, a rotary grinding apparatus, a method and an apparatus for obtaining a rotational grinding speed, a computer device, a storage medium, and a computer program product.

### BACKGROUND

A conventional rotational atherectomy catheter mainly includes a flexible drive shaft and a burr carried by the drive shaft and covered with a wear-resistant material such as diamond particles. The burr is driven by the drive shaft to rotate at a high speed, advance forward to contact a lesion, and abrade the lesion. Control of a rotational speed of the burr is therefore particularly important. In the related art, when the rotational speed is measured by an optical fiber sensor, an optical fiber probe is required to be disposed above a speed-measuring motor. The optical fiber probe rotates with the speed-measuring motor and passes by the optical fiber sensor twice in one revolution. Two square waves can thereby be obtained via the optical fiber sensor, and the rotational speed can be determined based on the obtained square waves.

However, during measurement of the rotational speed by the optical fiber sensor, the optical fiber probe is required to be disposed above the motor. The motor of a medical rotary grinding device is typically located inside a housing, and an interior space of the housing is typically narrow due to other precision components arranged therein, which makes it difficult to accommodate the optical fiber probe. In addition, for a medical rotary grinding device, disposing the optical fiber probe above the motor may interfere with rotation of the motor, thereby affecting operation accuracy.

### SUMMARY

Accordingly, there is a need to address the above technical problems by providing a rotary grinding device, a rotary grinding apparatus, a method for obtaining a rotational grinding speed, a rotational grinding catheter system, a computer device, a computer-readable storage medium, and a computer program product, which are capable of accurately obtaining a variation of a rotational grinding speed.

In a first aspect, a rotary grinding device is provided, including:
a drive shaft;
a rotating member sleeved over the drive shaft, the rotating member being provided with a trigger mark;
a detection member configured in response to triggering by the trigger mark to generate a trigger signal when the rotating member rotates with the drive shaft; and
a controller connected to the detection member and configured to determine a rotational speed of the drive shaft based on the trigger signal from the detection member.

In an embodiment, the detection member includes a light emitting module configured to emit an optical signal, and a light receiving module configured to receive the optical signal; an emission path of the light emitting module and a reception path of the light receiving module form an optical path; and during rotation of the rotating member, a trigger position of the trigger mark is on the optical path.

In an embodiment, the rotary grinding device further includes a motor, the motor includes a motor housing, the drive shaft is located within the motor housing, the motor housing is provided with an opening, and the light emitting module and the light receiving module are inserted into the motor housing via the opening.

In an embodiment, the trigger mark guides the optical path when the trigger mark is at the trigger position.

In an embodiment, an outer circumferential surface of the rotating member includes a light reflecting region and a light blocking region; and
the light reflecting region of the rotating member forms the trigger mark.

In an embodiment, at least a portion of a wall surface of the rotating member at the light reflecting region is configured as a light reflecting surface; or
at least a portion of the wall surface of the rotating member at the light reflecting region is provided with a light reflecting layer.

In an embodiment, at least a portion of a wall surface of the rotating member at the light blocking region is formed as a light blocking surface; or
at least a portion of the wall surface of the rotating member at the light-blocking region is provided with a light blocking layer.

In an embodiment, the rotating member is provided with a recess portion in the light-reflecting region, and an inner wall of the recess portion forms the trigger mark.

In an embodiment, the recess portion extends through an inner wall of the rotating member along a radial direction of the rotating member.

In a second aspect, a rotary grinding apparatus is provided, including a power source and the rotary grinding device according to any one of the first aspect. The power source is connected to the drive shaft for driving, and the power source is configured to drive the drive shaft to rotate.

In a third aspect, a method for obtaining a rotational grinding speed is provided. The method is applied to a rotary grinding device in which a rotating member is sleeved over a drive shaft, and the rotating member is provided with a trigger mark. The method includes:
obtaining corresponding trigger signals in response to triggering by the trigger mark during rotation of the rotating member with the drive shaft;
obtaining signal time differences each between two consecutive trigger signals; and
determining a rotational speed of the drive shaft based on all the signal time differences when the number of obtained signal time differences reaches a target number.

In an embodiment, the trigger signals are optical signals, and obtaining the signal time differences each between the two consecutive trigger signals includes:
converting the optical signals into voltage signals; and
amplifying the voltage signals when the voltage signals satisfy a voltage requirement condition, and obtaining the signal time differences each between two consecutive amplified voltage signals.

In an embodiment, determining the current rotational speed of the drive shaft based on all the signal time differences includes:
obtaining an average duration of all the signal time differences; and
determining the rotational speed of the drive shaft based on the average duration, wherein the average duration is a time required for one revolution of the drive shaft.

In an embodiment, the method further includes:
displaying a rotational speed waveform based on a plurality of rotational speeds, wherein the rotational speed waveform represents a variation trend of a rotational speed of a burr.

In a fourth aspect, an apparatus for obtaining a rotational grinding speed is provided, including:
a signal obtainment module configured to obtain corresponding trigger signals in response to triggering by a trigger mark during rotation of a rotating member with a drive shaft;
a time difference obtainment module configured to obtain signal time differences each between two consecutive trigger signals; and
a speed determination module configured to determine a rotational speed of the drive shaft based on all signal time differences when the number of obtained signal time differences reaches a target number.

In a fifth aspect, a computer device is provided, including a memory and a processor. The memory stores a computer program. The processor, when executing the computer program, implements steps of the method according to any one of the third aspect.

In a sixth aspect, a computer-readable storage medium is provided, having a computer program stored thereon. The computer program, when executed by a processor, implements steps of the method according to any one of the third aspect.

In a seventh aspect, a computer program product is provided, including a computer program. The computer program, when executed by a processor, implements steps of the method according to any one of the third aspect.

In the rotary grinding device, the rotary grinding apparatus, the method and apparatus for obtaining a rotational grinding speed, the computer device, the storage medium, and the computer program product described above, during rotation of the rotating member with the drive shaft, the trigger signal is generated by the trigger mark on the rotating member directly sleeved over the drive shaft. The signal time differences each between two consecutive trigger signals are obtained. When the number of obtained signal time differences reaches the target number, the rotational speed of the drive shaft is determined based on all the signal time differences, and the rotational speed waveform representing the variation trend of the rotational speed of the burr is displayed based on a plurality of rotational speeds. As such, no internal structural adjustment is required due to additional disposition of an optical fiber probe that would otherwise affect operation accuracy. Under cooperation of the detection member and the controller, rotational speed detection can be performed at any time, to accurately obtain a variation of the rotational grinding speed, thereby improving operational flexibility and usability.

### BRIEF DESCRIPTION OF DRAWINGS

For describing technical solutions in the embodiments of the present application or in the related art more clearly, the accompanying drawings used in describing the embodiments or the related art are briefly introduced below. Obviously, the drawings in the description below involve merely some embodiments of the present application, and other drawings can be obtained based on these drawings without inventive efforts by a person of ordinary skill in the art.
FIG. 1 is a schematic structural diagram of a rotary grinding device in an embodiment of the present application.
FIG. 2 is a schematic structural diagram of a rotating member in an embodiment of the present application.
FIG. 3 is a schematic structural diagram of a rotating member in another embodiment of the present application.
FIG. 4 is a schematic structural diagram of a rotary grinding apparatus in an embodiment of the present application.
FIG. 5 is a schematic diagram of a partial structure of a knob portion of a rotary grinding apparatus in an embodiment of the present application.
FIG. 6 is a schematic flowchart of a method for obtaining a rotational grinding speed in an embodiment of the present application.
FIG. 7 is a schematic diagram of a display screen in an embodiment of the present application.
FIG. 8 is a schematic flowchart of a method for obtaining a rotational grinding speed in an embodiment of the present application.
FIG. 9 is a schematic structural block diagram of an apparatus for obtaining a rotational grinding speed in an embodiment of the present application.
FIG. 10 is an internal structural diagram of a computer device in an embodiment of the present application.

Description of reference signs:
100: drive shaft;
200: rotating member, 210: light reflecting region, 211: recess portion, 220: light blocking region;
300: detection member, 310: light emitting module, 320: light receiving module;
400: controller;
500: motor, 510: motor housing, 511: opening, 512: gas inlet socket, 513: liquid inlet socket, 520: motor body;
600: advancer body, 610: advancer housing, 611: movement button, 612: gear button, 613: guidewire clamp, 614: wire-locking mechanism;
700: catheter assembly;
800: burr.

### DETAILED DESCRIPTION

To make the above objectives, features, and advantages of the present application more apparent and understandable, specific embodiments of the present application are described in detail below with reference to the accompanying drawings. Numerous specific details are set forth in the following description to facilitate a thorough understanding of the present application. However, the present application can be implemented in many other manners different from those described herein, and a person skilled in the art can make similar improvements without departing from the connotation of the present application. Therefore, the present application is not limited by the specific embodiments disclosed below.

In the description of the present application, it should be understood that when terms such as "center", "upper", "lower", "inner", "outer", "axial", and "radial" appear, an orientation or positional relationship indicated by these terms is based on the orientation or positional relationship shown in the accompanying drawings, and is merely for facilitating the description of the present application and simplifying the description, rather than indicating or implying that a referenced device or element must have a particular orientation or be constructed and operated in the particular orientation. Therefore, these terms cannot be construed as a limitation on the present application.

In the present application, unless otherwise expressly specified or defined, when terms such as "connect" and "fix" appear, these terms should be understood in a broad sense. For example, the connection may be a fixed connection, a detachable connection, or an integral connection. The connection may be a mechanical connection or an electrical connection. The connection may be a direct connection, an indirect connection through an intermediate medium, an internal communication within two elements, or an interaction relationship between two elements, unless otherwise expressly defined. For a person of ordinary skill in the art, specific meanings of the above terms in the present application can be understood according to specific situations.

It should be noted that when an element is referred to as being "fixed to" another element, the element may be directly on the other element, or there may be an intermediate element therebetween. When an element is referred to as being "connected to" another element, the element may be directly connected to the other element, or there may be an intermediate element therebetween. The terms "upper", "lower", and similar expressions used in the present application, if any, are merely for the purpose of illustration and do not represent the only embodiment.

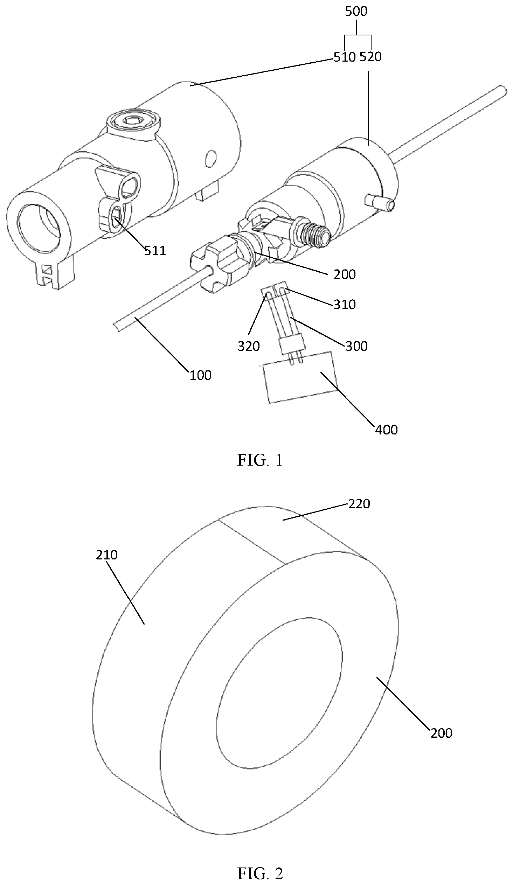

In an exemplary embodiment, as shown in FIG. 1, which is a schematic structural diagram of a rotary grinding device provided in an embodiment of the present application. The rotary grinding device includes a drive shaft 100, a rotating member 200, a detection member 300, and a controller 400. The rotating member 200 is sleeved over the drive shaft 100 and rotates synchronously with rotation of the drive shaft 100, and the rotating member 200 is provided with a trigger mark. The detection member 300 is disposed opposite the rotating member 200, and the detection member 300 is configured in response to triggering by the trigger mark to generate a trigger signal when the rotating member 200 rotates with the drive shaft 100. The controller 400 is connected to the detection member 300, and the controller 400 is configured to determine a rotational speed of the drive shaft 100 based on the trigger signal from the detection member 300.

In the rotary grinding device provided in an embodiment of the present application, since the trigger mark can be provided directly on the rotating member sleeved over the drive shaft, no optical fiber probe is required to be additionally provided, and therefore no internal structural adjustment is required due to disposition of the optical fiber probe that would otherwise affect operation accuracy. In addition, since the trigger mark is provided on the existing rotating member rather than using an optical fiber probe, rotational speed detection can be achieved, and therefore costs can be reduced. Finally, since the trigger mark is provided on the rotating member, rotational speed detection can be performed at any time through cooperation of the detection member and the controller. The detection member and the controller themselves are not required to be integrated as a single unit with a rotary grinding apparatus, that is, when rotational speed detection is not required, the detection member and the controller can be removed from the rotary grinding apparatus, thereby improving operational flexibility and usability.

In an embodiment, with continued reference to FIG. 1, the detection member 300 includes a light emitting module 310 configured to emit an optical signal, and a light receiving module 320 configured to receive the optical signal. An emission path of the light emitting module 310 and a reception path of the light receiving module 320 form an optical path. During rotation of the rotating member 200, a trigger position of the trigger mark is on the optical path.

In this embodiment, the detection member can include the light emitting module and the light receiving module, and the trigger mark can be provided at the trigger position on the optical path, so that rotational speed detection can be achieved through the photoemission, such that the detection process has a wider measurement range, a shorter measurement duration, and a higher measurement accuracy.

In an embodiment, as shown in FIG. 1, the rotary grinding device further includes a motor 500. The motor 500 includes a motor housing 510 and a motor body 520. The drive shaft 100 is located within the motor housing 510. A surface of the motor housing 510 is provided with an opening 511. The light emitting module 310 and the light receiving module 320 are inserted into the motor housing 510 via the opening 511.

The opening 511 is designed to cover a region where the trigger mark is located. It can be understood that, as shown in FIG. 1, the light emitting module 310 and the light receiving module 320 are inserted into the motor housing 510 via the opening 511 and are disposed directly facing the rotating member 200.

In this embodiment, by providing the opening on the motor housing, the light emitting module and the light receiving module can be inserted into the motor via the opening. Since the opening is provided on the motor housing, the optical path can be established inside the motor without providing an additional path channel, and therefore costs can be reduced.

In an embodiment, the trigger mark guides the optical path when the trigger mark is at the trigger position. The trigger position refers to a position where the trigger mark is within a detection range of the light emitting module 310 and the light receiving module 320. During rotation of the rotating member 200 with the drive shaft 100 and when the trigger mark rotates with the rotating member 200 to the trigger position, since the light emitting module 310 and the light receiving module 320 are disposed directly facing the rotating member 200, the light emitting module 310 constantly transmits the optical signal to the rotating member 200. Only when the trigger mark rotates to the trigger position is the optical path guided. In this case, the light emitting module 310 transmits the optical signal to the trigger mark, and at the same time, the light receiving module 320 obtains the trigger signal of the rotating member 200 in response to triggering by the trigger mark.

In this embodiment, since the trigger mark can be provided directly on the rotating member sleeved over the drive shaft, no internal structural adjustment is required due to disposition of the optical fiber probe that would otherwise affect operation accuracy. In addition, since the trigger mark is provided on the existing rotating member rather than using an optical fiber probe, rotational speed detection can be achieved, and therefore, the costs can be reduced.

In an embodiment, as shown in FIG. 2, which is a schematic structural diagram of the rotating member 200, an outer circumferential surface of the rotating member 200 includes a light reflecting region 210 and a light blocking region 220, and the light reflecting region 210 of the rotating member 200 forms the trigger mark.

The light reflecting region 210 refers to a region capable of transmitting the optical signal. The light blocking region 220 refers to a region that does not transmit the optical signal. When the light emitting module 310 transmits the optical signal to the rotating member 200, and when the light blocking region 220 rotates to face the light receiving module 320, the optical signal is absorbed, so that the light receiving module 320 cannot receive the transmitted optical signal. Only when the light reflecting region 210 rotates to face the light receiving module 320, the optical signal is transmitted to the light receiving module 320 through the light reflecting region 210, and the trigger signal is generated. To ensure accuracy of the trigger signal, a size of the light reflecting region 210 should generally be smaller than that of the light blocking region 220. When the light reflecting region 210 is set too large, a transmission duration of the optical signal is too long, which may cause the light receiving module 320 to fail to receive a complete trigger signal.

In this embodiment, the light reflecting region and the light blocking region can be provided on the outer circumferential surface of the rotating member. Since the light reflecting region can form the trigger mark, and the light blocking region can block light propagation to reduce interference with the trigger mark, measurement accuracy can be improved.

In an embodiment, with reference to FIG. 2, at least a portion of a wall surface of the rotating member 200 at the light reflecting region 210 is formed as a light reflecting surface, or at least a portion of the wall surface of the rotating member 200 at the light reflecting region 210 is provided with a light reflecting layer. The light reflecting region 210 may be a light reflecting surface formed by electroplating or the like, or a light reflecting layer formed on the wall surface of the light reflecting region 210 by using a material capable of transmitting the optical signal. When the rotating member 200 rotates to the trigger position, the light receiving module 320 receives the optical signal transmitted by the light reflecting region 210, thereby collecting the trigger signal.

In this embodiment, since a light reflecting surface or a light reflecting layer can be formed on at least a portion of the wall surface of the light reflecting region, no other component is required to be additionally used to form the light reflecting region. Therefore, no extensive adjustment to an internal structure of the rotary grinding device is required, and therefore, costs of modification and use can be reduced.

In an embodiment, at least a portion of a wall surface of the rotating member 200 at the light blocking region 220 is formed as a light blocking surface, or at least a portion of the wall surface of the rotating member 200 at the light blocking region 220 is provided with a light blocking layer. The light blocking region 220 may be a light blocking surface formed by oxidation or the like, or a light blocking layer formed on the wall surface of the light blocking region 220 by using a material that does not transmit the optical signal. Since the light blocking region 220 does not transmit the optical signal, when the light blocking region 220 rotates to the trigger position, the optical signal transmitted by the light emitting module 310 is absorbed, and the light receiving module 320 does not receive the transmitted optical signal.

In this embodiment, since at least a portion of the wall surface of the light blocking region can be formed as a light blocking surface or a light blocking layer, no other component is required to be additionally used to form the light blocking region. Therefore, no extensive adjustment to the internal structure of the rotary grinding device is required, and therefore, the costs of modification and use can be reduced.

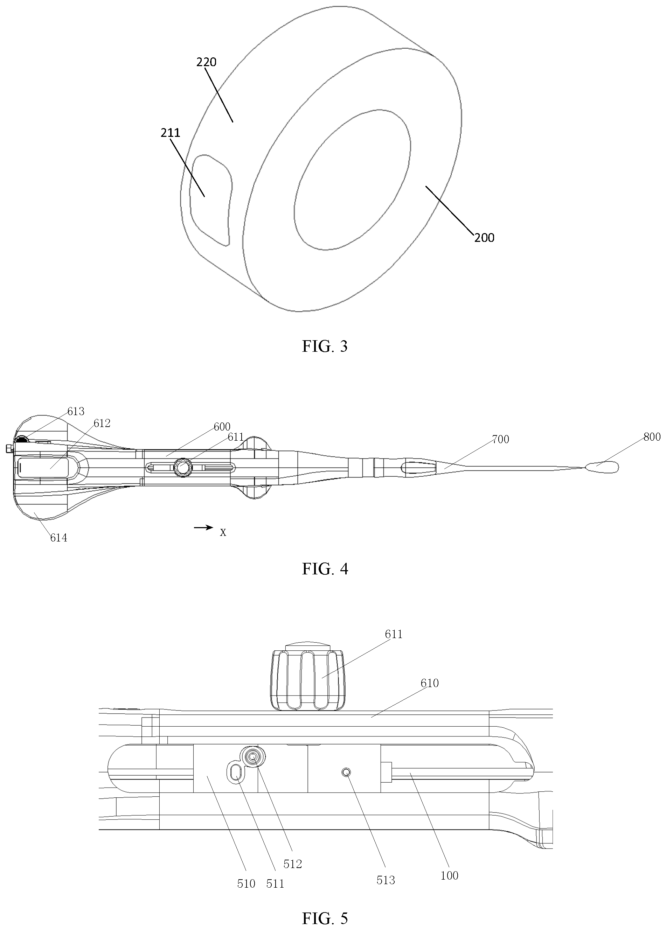

In an embodiment, as shown in FIG. 3, which is another schematic structural diagram of the rotating member 200, the rotating member 200 is provided with a recess portion 211 in the light reflecting region 210, and an inner wall of the recess portion 211 forms the trigger mark.

Specifically, the inner wall of the recess portion 211 can form the trigger mark by embedding a metal component or the like. Further, the recess portion 211 is located on an outer surface of the rotating member 200. During rotation of the rotating member 200, the recess portion 211 rotates with the rotating member 200 until the recess portion 211 rotates to the trigger position, in which case the light receiving module 320 receives the optical signal transmitted by the trigger mark formed by the inner wall of the recess portion 211. It can be understood that the specific manner of forming the light reflecting region 210 and the light blocking region 220 in the rotating member 200 provided in an embodiment of the present application is not limited, as long as the trigger mark can be formed.

In this embodiment, the recess portion is provided on the rotating member, and the trigger mark is formed by the inner wall of the recess portion. Since only the rotating member itself needs to be adjusted, no extensive adjustment to the internal structure of the rotary grinding apparatus is required, and therefore, the costs of modification and use can be reduced.

In an embodiment, the recess portion 211 extends through an inner wall of the rotating member 200 along a radial direction of the rotating member 200. Since the rotating member 200 is sleeved over the drive shaft 100, and an outer surface of the drive shaft 100 is generally made of a metal material, the recess portion 211 is configured to extend through the inner wall of the rotating member 200 along the radial direction of the rotating member 200, so that the outer surface of the drive shaft 100 is exposed through the recess portion 211. When the recess portion 211 rotates to the trigger position, the optical signal is transmitted through the exposed portion of the drive shaft 100. In other embodiments, the recess portion 211 may be a slot opened along the radial direction of the rotating member 200 without extending through the inner wall of the rotating member 200, and the light reflecting region 210 may be formed by a light reflecting layer provided at a bottom of the slot.

In this embodiment, by extending the recess portion through the inner wall of the rotating member along the radial direction, a metal outer wall of the drive shaft can be exposed to serve as the light reflecting region. Since only the rotating member itself needs to be adjusted, no additional component for forming the light reflecting region needs to be used, and thereby the costs of modification and use can be reduced.

In an embodiment, as shown in FIG. 4 to FIG. 5, a rotary grinding apparatus is provided. The rotary grinding apparatus includes the rotary grinding device in the above embodiments, and the rotary grinding apparatus further includes an advancer body 600, a catheter assembly 700, and a burr 800. One end of the advancer body 600 along an x direction is connected to the burr 800 through the catheter assembly 700, one end of the detection member 300 is inserted into the advancer body 600, and the other end of the detection member 300 is connected to the controller 400. It can be understood that FIG. 5 shows a schematic connection of the controller 400, and the controller 400 may be any processor having an embedded algorithm, such as a terminal. In an embodiment, the rotary grinding apparatus further includes a power source, and the power source is connected to the drive shaft for driving and is configured to drive the drive shaft of the rotary grinding device to rotate.

Specifically, the advancer body 600 includes an advancer housing 610, and the drive shaft 100 and the motor 500 are arranged inside the advancer housing 610. The drive shaft 100 is connected to the catheter assembly 700 and is configured to be rotatable under driving of the motor 500, thereby driving the burr 800 to rotate. A surface of the advancer housing 610 is provided with a movement button 611 and a gear button 612. The movement button 611 is located in a groove on the surface of the advancer housing 610 and moves along the x direction. The movement button 611 is connected to the motor 500 and is configured to drive, via the motor 500, the drive shaft 100 to move when moving along the x direction, so as to drive, via the catheter assembly 700, the burr 800 to extend or retract. The gear button 612 is located near the other end of the advancer body 600 opposite along the x direction. A guidewire clamp 613 and a wire-locking mechanism 614 are further provided at the other end of the advancer housing 610 opposite along the x direction. The x direction is a direction along a central axis of the advancer body 600 toward the burr 800. In an embodiment, a side surface of the advancer body 600 is provided with an opening that communicates with the interior of the advancer housing 610, and a size of the opening covers the groove on the surface of the advancer housing 610. In an embodiment, the motor housing 510 is further provided with a gas inlet socket 512 and a liquid inlet socket 513.

In the rotary grinding device provided in an embodiment of the present application, since the trigger mark can be provided directly on the rotating member sleeved over the drive shaft, no optical fiber probe is required to be additionally provided, and therefore no internal structural adjustment is required due to disposition of the optical fiber probe that would otherwise affect operation accuracy. In addition, since the trigger mark is provided on the existing rotating member rather than using an optical fiber probe, rotational speed detection can be achieved, and therefore costs can be reduced. Finally, since the trigger mark is provided on the rotating member, rotational speed detection can be performed at any time under cooperation of the detection member and the controller. The detection member and the controller themselves are not required to be integrated as a single unit with the rotary grinding apparatus, that is, when rotational speed detection is not required, the detection member and the controller can be removed from the rotary grinding apparatus, thereby improving operational flexibility and usability.

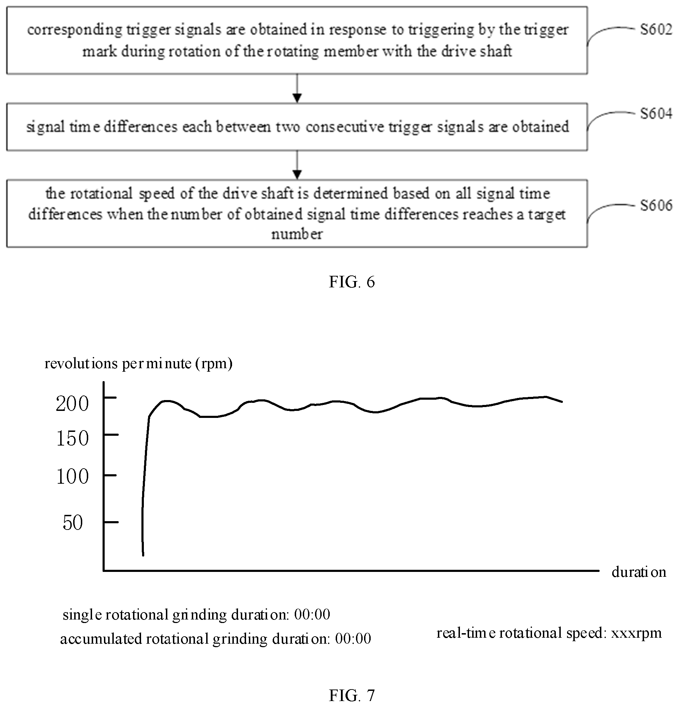

In an exemplary embodiment, as shown in FIG. 6, a method for obtaining a rotational grinding speed is provided. The method is described by taking an example in which the method is applied to the controller 400. The method includes the following Step S602 to Step S606.

At Step S602, corresponding trigger signals are obtained in response to triggering by the trigger mark during rotation of the rotating member with the drive shaft.

Specifically, the rotating member includes the light reflecting region and the light blocking region, and the light reflecting region forms the trigger mark. During rotation of the rotating member with the drive shaft, the controller emits the optical signal to the rotating member through the light emitting module in the detection member. When the optical signal is transmitted to the light reflecting region, the light receiving module receives the optical signal reflected back by the light reflecting region, that is, the corresponding trigger signal is received in response to triggering by the trigger mark. When the optical signal is transmitted to the light blocking region, the light receiving module is not triggered.

At Step S604, signal time differences each between two consecutive trigger signals are obtained.

Specifically, a reception frequency of the trigger signal is related to the rotational speed of the drive shaft. Therefore, the signal time difference between two consecutive trigger signals is determined based on a reception time of the trigger signal. The signal time difference is a time required for one rotation of the drive shaft, that is, a time required for one rotation of the burr of the rotary grinding device.

At Step S606, the rotational speed of the drive shaft is determined based on all the signal time differences when the number of obtained signal time differences reaches a target number.

Specifically, since the rotational speed of the drive shaft is relatively high, the obtained signal time difference is generally relatively short. To ensure accuracy of the rotational speed, a sufficient number of signal time differences need to be obtained. When the number of obtained signal time differences reaches the target number, the signal time differences are averaged to obtain the rotational speed of the drive shaft within this period of time, that is, the rotational speed of the burr.

In the above method for obtaining the rotational grinding speed, during rotation of the rotating member with the drive shaft, the trigger signal is generated by the trigger mark on the rotating member directly sleeved over the drive shaft. The signal time differences each between two consecutive trigger signals are obtained. When the number of obtained signal time differences reaches the target number, the rotational speed of the drive shaft is determined based on all the signal time differences, and a rotational speed waveform representing a variation trend of the rotational speed of the burr is displayed based on a plurality of rotational speeds. As such, no internal structural adjustment is required due to additional disposition of an optical fiber probe that would otherwise affect operation accuracy. Through cooperation of the detection member and the controller, rotational speed detection can be performed at any time, to accurately obtain a variation of the rotational grinding speed, thereby improving operational flexibility and usability.

In an exemplary embodiment, the trigger signals are optical signals, and the step of obtaining the signal time differences each between the two consecutive trigger signals can include: converting the optical signals into voltage signals; amplifying the voltage signals when the voltage signals satisfy a voltage requirement condition; and obtaining the signal time differences each between two consecutive amplified voltage signals.

Specifically, a signal that the controller can process is a voltage signal of 2 V to 3.3 V. The optical signal first needs to be converted into a voltage signal, and the voltage signal at this time is a voltage of 0 V to 0.8 V. Since the optical signal is greatly affected by the environment, the voltage may be too small when the optical signal is converted into the voltage signal. Therefore, the voltage signal is determined. When the voltage signal satisfies the voltage requirement condition, the voltage signal is valid. In this case, the voltage signal is amplified to a voltage range that the controller can process, and the controller obtains the signal time difference between two consecutive amplified voltage signals. The voltage requirement condition is that the voltage is greater than 0.5 V.

In this embodiment, by converting the optical signals into voltage signals, amplifying the voltage signals when the voltage signals satisfy the voltage requirement condition, and obtaining the signal time differences each between two consecutive amplified voltage signals, validity of the voltage signal can be ensured, so that the rotational grinding speed is accurately obtained.

In an exemplary embodiment, the step of determining the current rotational speed of the drive shaft based on all the signal time differences can include: obtaining an average duration of all the signal time differences; and determining the rotational speed of the drive shaft based on the average duration, where the average duration is a time required for one rotation of the drive shaft.

Specifically, when the number of obtained signal time differences reaches the target number, the controller performs averaging to obtain the average duration corresponding to all the signal time differences, where the average duration is a time required for one rotation of the drive shaft. The rotational speed of the drive shaft is determined by calculating the average duration.

In this embodiment, by obtaining the average duration of all the signal time differences and determining the rotational speed of the drive shaft based on the average duration, where the average duration is a time required for one rotation of the drive shaft, accuracy of the rotational speed can be ensured.

In an exemplary embodiment, the method further includes: displaying the rotational speed waveform based on a plurality of rotational speeds, where the rotational speed waveform represents the variation trend of the rotational speed of the burr.

Specifically, each time the target number of signal time differences is obtained, the controller calculates the rotational speed within this period of time, so as to obtain a plurality of rotational speeds varying over time. In addition, the controller is connected to a display screen through an external wire and sends the rotational speeds to the display screen, to display the rotational speed waveform representing the variation trend of the rotational speed of the burr. In an embodiment, as shown in FIG. 7, which is a schematic diagram of the display screen displaying the rotational speed waveform, a horizontal axis of the rotational speed waveform represents time, and a vertical axis represents a rotational speed (rpm). Meanwhile, the display screen further displays, for each driving of the drive shaft, a single rotational grinding duration and an accumulated rotational grinding duration, as well as a real-time rotational speed.

In this embodiment, by displaying the rotational speed waveform representing the variation trend of the rotational speed of the burr based on a plurality of rotational speeds, a variation of the rotational speed of the burr can be obtained in real time.

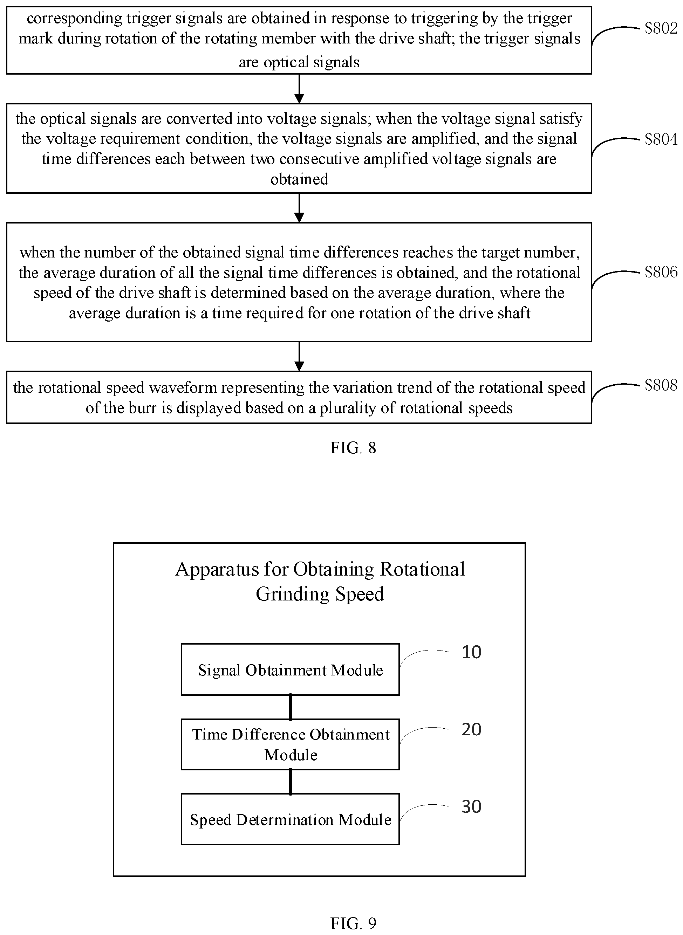

In an exemplary embodiment, as shown in FIG. 8, a method for obtaining a rotational grinding speed is provided, and the method includes the following steps.

At Step S802, corresponding trigger signals are obtained in response to triggering by the trigger mark during rotation of the rotating member with the drive shaft.

At Step S804, the optical signals are converted into voltage signals; when the voltage signals satisfy the voltage requirement condition, the voltage signals are amplified, and signal time differences each between two consecutive amplified voltage signals are obtained.

At Step S806, when the number of obtained signal time differences reaches the target number, the average duration of all the signal time differences is obtained, and the rotational speed of the drive shaft is determined based on the average duration, where the average duration is a time required for one rotation of the drive shaft.

At Step S808, the rotational speed waveform representing the variation trend of the rotational speed of the burr is displayed based on a plurality of rotational speeds.

In this embodiment, by obtaining corresponding trigger signals in response to triggering by the trigger mark during rotation of the rotating member with the drive shaft, obtaining the signal time differences each between two consecutive trigger signals, determining the rotational speed of the drive shaft based on all the signal time differences when the number of obtained signal time differences reaches the target number, and displaying the rotational speed waveform representing the variation trend of the rotational speed of the burr based on a plurality of rotational speeds, a variation of the rotational grinding speed can be accurately obtained based on the rotational speed waveform.

It should be understood that although the steps in the flowcharts involved in the above embodiments are displayed sequentially as indicated by arrows, these steps are not necessarily performed sequentially in the order indicated by the arrows. Unless expressly stated herein, there is no strict order limitation on the performance of these steps, and these steps can be performed in other orders. Moreover, at least a portion of the steps in the flowcharts involved in the above embodiments can include a plurality of steps or a plurality of stages. These steps or stages are not necessarily performed and completed at the same moment, but can be performed at different moments. The order of performing these steps or stages is not necessarily sequential either, but these steps or stages can be performed alternately or in turn with at least a portion of other steps or at least a portion of steps or stages within other steps.

Based on the same inventive concept, an embodiment of the present application further provides an apparatus for obtaining a rotational grinding speed for implementing the above method for obtaining the rotational grinding speed. An implementation provided by the apparatus for solving the problem is similar to that described in the above method. Therefore, for specific definitions in one or more embodiments of the apparatus for obtaining a rotational grinding speed provided below, reference can be made to the definitions of the method for obtaining the rotational grinding speed above, and details are not described herein again.

In an exemplary embodiment, as shown in FIG. 9, an apparatus for obtaining a rotational grinding speed is provided, including a signal obtainment module 10, a time difference obtainment module 20, and a speed determination module 30.

The signal obtainment module 10 is configured to obtain corresponding trigger signals in response to triggering by the trigger mark during rotation of the rotating member with the drive shaft.

The time difference obtainment module 20 is configured to obtain the signal time differences each between two consecutive trigger signals.

The speed determination module 30 is configured to determine the rotational speed of the drive shaft based on all signal time differences when the number of obtained signal time differences reaches the target number.

In an exemplary embodiment, the time difference obtainment module 20 is further configured to convert the optical signals into voltage signals, amplify the voltage signals when the voltage signals satisfy the voltage requirement condition, and obtain the signal time differences each between two consecutive amplified voltage signals.

In an exemplary embodiment, the speed determination module 30 is further configured to obtain the average duration of all the signal time differences and determine the rotational speed of the drive shaft based on the average duration, where the average duration is a time required for one rotation of the drive shaft.

In an exemplary embodiment, the speed determination module 30 is further configured to display the rotational speed waveform representing the variation trend of the rotational speed of the burr based on a plurality of rotational speeds.

All or a portion of the modules in the above apparatus for obtaining the rotational grinding speed can be implemented by software, hardware, or a combination thereof. The above modules can be embedded in or independent of a processor in a computer device in a hardware form, or can be stored in a memory in the computer device in a software form, so that the processor invokes and performs operations corresponding to the above modules.

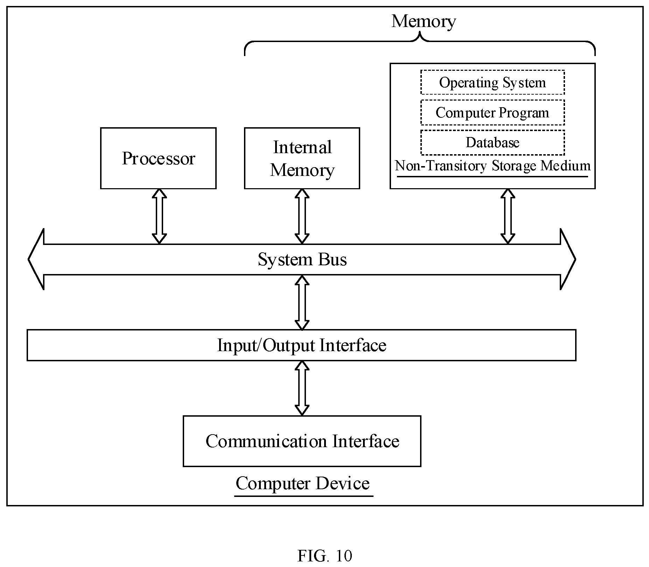

In an exemplary embodiment, a computer device is provided. The computer device may be a terminal, and an internal structural diagram thereof may be as shown in FIG. 10. The computer device includes a processor, a memory, an input/output interface, a communication interface, a display unit, and an input device. The processor, the memory, and the input/output interface are connected through a system bus, and the communication interface, the display unit, and the input device are connected to the system bus through the input/output interface. The processor of the computer device is configured to provide computing and control capabilities. The memory of the computer device includes a non-volatile storage medium and an internal memory. The non-volatile storage medium stores an operating system and a computer program. The internal memory provides an environment for running the operating system and the computer program in the non-volatile storage medium. The input/output interface of the computer device is configured to exchange information between the processor and an external device. The communication interface of the computer device is configured to communicate with an external terminal in a wired or wireless manner, and the wireless manner can be implemented by WIFI, a mobile cellular network, NFC (near field communication), or other techniques. The computer program, when executed by the processor, implements a method for obtaining a rotational grinding speed. The display unit of the computer device is configured to form a visually visible picture, and may be a display screen, a projection device, or a virtual reality imaging device. The display screen may be a liquid crystal display screen or an electronic ink display screen. The input device of the computer device may be a touch layer covering the display screen, may be a button, a trackball, or a touchpad provided on a housing of the computer device, or may be an external keyboard, touchpad, mouse, or the like.

A person skilled in the art can understand that the structure shown in FIG. 10 is merely a block diagram of a part of the structure related to the solution of the present application, and does not constitute a limitation on the computer device to which the solution of the present application is applied. A specific computer device can include more or fewer components than those shown in the figure, combine some components, or have a different component arrangement.

In an exemplary embodiment, a computer device is provided, including a memory and a processor. The memory stores a computer program. The processor, when executing the computer program, implements the following steps: obtaining corresponding trigger signals in response to triggering by the trigger mark during rotation of the rotating member with the drive shaft; obtaining the signal time differences each between two consecutive trigger signals; and determining the rotational speed of the drive shaft based on all the signal time differences when the number of obtained signal time differences reaches the target number.

In an embodiment, the trigger signals are optical signals, and when executing the computer program, the processor further implements the following steps: converting the optical signals into voltage signals; amplifying the voltage signals when the voltage signals satisfy the voltage requirement condition; and obtaining the signal time differences each between two consecutive amplified voltage signals.

In an embodiment, when executing the computer program, the processor further implements the following steps: obtaining the average duration of all the signal time differences; and determining the rotational speed of the drive shaft based on the average duration, where the average duration is a time required for one rotation of the drive shaft.

In an embodiment, when executing the computer program, the processor further implements the following step: displaying the rotational speed waveform representing the variation trend of the rotational speed of the burr based on a plurality of rotational speeds.

In an embodiment of the present application, a computer-readable storage medium is further provided, having a computer program stored thereon. The computer program, when executed by a processor, implements the following steps: obtaining corresponding trigger signals in response to triggering by the trigger mark during rotation of the rotating member with the drive shaft; obtaining the signal time differences each between two consecutive trigger signals; and determining the rotational speed of the drive shaft based on all the signal time differences when the number of obtained signal time differences reaches the target number.

In an embodiment, the trigger signals are optical signals, and when executed by the processor, the computer program further implements the following steps: converting the optical signals into voltage signals; amplifying the voltage signals when the voltage signals satisfy the voltage requirement condition; and obtaining the signal time differences each between two consecutive amplified voltage signals.

In an embodiment, when executed by the processor, the computer program further implements the following steps: obtaining the average duration corresponding to all the signal time differences; and determining the rotational speed of the drive shaft based on the average duration, where the average duration is a time required for one rotation of the drive shaft.

In an embodiment, when executed by the processor, the computer program further implements the following step: displaying the rotational speed waveform representing the variation trend of the rotational speed of the burr based on a plurality of rotational speeds.

In an embodiment of the present application, a computer program product is further provided, including a computer program. The computer program, when executed by a processor, implements the following steps: obtaining corresponding trigger signals in response to triggering by the trigger mark during rotation of the rotating member with the drive shaft; obtaining the signal time differences each between two consecutive trigger signals; and determining the rotational speed of the drive shaft based on all the signal time differences when the number of obtained signal time differences reaches the target number.

In an embodiment, the trigger signals are optical signals, and when executed by the processor, the computer program further implements the following steps: converting the optical signals into voltage signals; amplifying the voltage signals when the voltage signals satisfy the voltage requirement condition; and obtaining the signal time differences each between two consecutive amplified voltage signals.

In an embodiment, when executed by the processor, the computer program further implements the following steps: obtaining the average duration corresponding to all the signal time differences; and determining the rotational speed of the drive shaft based on the average duration, where the average duration is a time required for one rotation of the drive shaft.

In an embodiment, when executed by the processor, the computer program further implements the following step: displaying the rotational speed waveform representing the variation trend of the rotational speed of the burr based on a plurality of rotational speeds.

A person of ordinary skill in the art can understand that all or a portion of the processes for implementing the methods in the above embodiments can be completed by a computer program instructing relevant hardware. The computer program can be stored in a non-volatile computer-readable storage medium. When executed, the computer program can include the processes of the embodiments of the above methods. Any reference to a memory, a database, or another medium used in the embodiments provided in the present application can include at least one of a non-volatile memory and a volatile memory. The non-volatile memory can include a read-only memory (ROM), a magnetic tape, a floppy disk, a flash memory, an optical memory, a high-density embedded non-volatile memory, a resistive random access memory (ReRAM), a magnetoresistive random access memory (MRAM), a ferroelectric random access memory (FRAM), a phase change memory (PCM), a graphene memory, etc. The volatile memory can include a random access memory (RAM), an external cache memory, etc. By way of illustration and not limitation, the RAM may be in various forms, such as a static random access memory (SRAM) or a dynamic random access memory (DRAM). A database involved in the embodiments provided in the present application can include at least one of a relational database and a non-relational database. The non-relational database can include a blockchain-based distributed database, etc., without being limited thereto. A processor involved in the embodiments provided in the present application can be a general-purpose processor, a central processing unit, a graphics processing unit, a digital signal processor, a programmable logic device, a quantum-computing-based data processing logic device, etc., without being limited thereto.

The technical features of the above embodiments can be combined arbitrarily. For brevity of description, not all possible combinations of the technical features in the above embodiments are described. However, as long as there is no contradiction in the combinations of these technical features, the combinations should be considered as falling within the scope described in this specification.

The above embodiments merely express several implementations of the present application, and descriptions thereof are relatively specific and detailed, but they should not be construed as a limitation on the patent scope of the present application. It should be noted that, for a person of ordinary skill in the art, several variations and improvements can be made without departing from the concept of the present application, and these all fall within the protection scope of the present application. Therefore, the protection scope of the present application shall be subject to the appended claims.

## Claims

1. A rotary grinding device, comprising:
a drive shaft;
a rotating member, sleeved over the drive shaft and provided with a trigger mark;
a detection member, configured in response to triggering by the trigger mark to generate a trigger signal when the rotating member rotates with the drive shaft; and
a controller, connected to the detection member and configured to determine a rotational speed of the drive shaft based on the trigger signal from the detection member.

2. The rotary grinding device according to claim 1, wherein the detection member comprises a light emitting module configured to emit an optical signal, and a light receiving module configured to receive the optical signal;
an emission path of the light emitting module and a reception path of the light receiving module form an optical path; and
during rotation of the rotating member, a trigger position of the trigger mark is on the optical path.

3. The rotary grinding device according to claim 2, further comprising a motor, wherein the motor comprises a motor housing, the drive shaft is located within the motor housing, the motor housing is provided with an opening, and the light emitting module and the light receiving module are inserted into the motor housing via the opening.

4. The rotary grinding device according to claim 2, wherein the trigger mark guides the optical path when the trigger mark is at the trigger position.

5. The rotary grinding device according to claim 4, wherein an outer circumferential surface of the rotating member comprises a light reflecting region and a light blocking region; and
the light reflecting region of the rotating member forms the trigger mark.

6. The rotary grinding device according to claim 5, wherein at least a portion of a wall surface of the rotating member at the light reflecting region is configured as a light reflecting surface; or
at least a portion of the wall surface of the rotating member at the light reflecting region is provided with a light reflecting layer.

7. The rotary grinding device according to claim 5, wherein at least a portion of a wall surface of the rotating member at the light blocking region is formed as a light blocking surface; or
at least a portion of the wall surface of the rotating member at the light blocking region is provided with a light blocking layer.

8. The rotary grinding device according to claim 5, wherein the rotating member is provided with a recess portion in the light reflecting region, and an inner wall of the recess portion forms the trigger mark.

9. The rotary grinding device according to claim 8, wherein the recess portion extends through an inner wall of the rotating member along a radial direction of the rotating member.

10. A rotary grinding apparatus, comprising a power source and the rotary grinding device according to any one of claims 1 to 9, wherein the power source is connected to the drive shaft for driving, and the power source is configured to drive the drive shaft to rotate.

11. A method for obtaining a rotational grinding speed, applied to a rotary grinding device including a drive shaft and a rotating member sleeved over the drive shaft, the rotating member being provided with a trigger mark, and the method comprising:
obtaining corresponding trigger signals in response to triggering by the trigger mark during rotation of the rotating member with the drive shaft;
obtaining signal time differences each between two consecutive trigger signals; and
determining a rotational speed of the drive shaft based on all the signal time differences when the number of the obtained signal time differences reaches a target number.

12. The method according to claim 11, wherein the trigger signals are optical signals, and obtaining the signal time differences each between the two consecutive trigger signals comprises:
converting the optical signals into voltage signals;
amplifying the voltage signals when the voltage signals satisfy a voltage requirement condition; and
obtaining the signal time differences each between two consecutive amplified voltage signals.

13. The method according to claim 11, wherein determining the current rotational speed of the drive shaft based on all the signal time differences comprises:
obtaining an average duration of all the signal time differences; and
determining the rotational speed of the drive shaft based on the average duration, wherein the average duration is a time required for one rotation of the drive shaft.

14. The method according to claim 11, further comprising:
displaying a rotational speed waveform based on a plurality of rotational speeds, wherein the rotational speed waveform represents a variation trend of a rotational speed of a burr.

15. An apparatus for obtaining a rotational grinding speed, comprising:
a signal obtainment module, configured to obtain corresponding trigger signals in response to triggering by a trigger mark during rotation of a rotating member with a drive shaft, wherein the rotating member is sleeved over the drive shaft and is provided with the trigger mark;
a time difference obtainment module, configured to obtain signal time differences each between two consecutive trigger signals; and
a speed determination module, configured to determine a rotational speed of the drive shaft based on all signal time differences when the number of obtained signal time differences reaches a target number.

16. A computer-readable storage medium, having a computer program stored thereon, the computer program, when executed by a processor, implements steps of the method according to any one of claims 11 to 14.

17. A computer program product, comprising a computer program, the computer program, when executed by a processor, implements steps of the method according to any one of claims 11 to 14.
